# FASCICULE DE BREVET EUROPEEN

(11) **EP 0 390 716 B1**
(45) Date de publication et mention de la délivrance du brevet: **15.12.1993**
(21) Numéro de dépôt: 90460018.6
(22) Date de dépôt: 28.03.1990
(51) Int. Cl.: C12N 1/06

(54) **Procédé de préparation de lysats de levure**
Verfahren zur Herstellung von Hefelysaten
Method for producing yeast lysates

(30) Priorité: 29.03.1989 FR 8904341
(43) Date de publication de la demande: 03.10.1990
(73) Titulaire: LA PROBIOLYSE, F-22100 Saint Samson sur Rance (FR)
(72) Inventeur: Cohas, Pascal, F-53000 Laval (FR); Cohas, Patrice, F-35630 Hede (FR)
(74) Mandataire: Le Guen, Louis François

(56) Documents cités:
- EP-A- 0 039 415
- EP-A- 0 234 863
- EP-A- 0 249 435
- DE-A- 2 205 464
- GB-A- 952 713
- PATENT ABSTRACTS OF JAPAN, vol. 11, no. 80 (C-409), 11 mars 1987

## Description

La présente invention concerne un procédé de préparation de lysats de levures de souche "Saccharomyces cerevisiae", ou de souche "Saccharomyces carlsbergensis" ou d'un mélange de ces deux souches.

Les lysats de levures sont utilisables, en particulier, comme additifs dans l'alimentation de nombreux animaux chez qui ils sont un facteur de croissance et ont un effet régulateur sur la flore bactérienne intestinale.

Le procédé de préparation suivant l'invention a pour objet d'obtenir de tels lysats en partant aussi bien de la levure de bière de souche "Saccharomyces carlsbergensis" que de la levure de panification de souche "Saccharomyces cerevisiae". En effet, on connaît déjà des procédés de préparation d'additifs nutritifs en partant de la levure de panification, mais jusqu'ici ces procédés ne permettaient pas l'utilisation de la levure de bière. L'utilisation de celle-ci est d'une grande importance car la levure de bière est beaucoup moins chère que la levure de panification et il en existe de grande quantité dans les brasseries où elle constitue un sous-produit de la fermentation.

Suivant une caractéristique de l'invention, il est prévu un procédé de préparation, tel que mentionné ci-dessus, dans lequel on fait subir aux levures un traitement physico-chimique d'éclatement de la paroi des cellules de levure en chauffant à une température supérieure à 72° C pendant au moins 20 minutes en présence de chlorure de sodium mélangé à la levure à raison de 2,6 à 10 % de chlorure de sodium, puis un second traitement dans lequel le lysat de cellules éclatées et salées de levures est additionné de 2,6 à 10 % d'un sel ou d'un mélange de sels phosphoriques ou phosphoreux, ou d'acide ou d'anhydrique phosphorique ou phosphoreux.

Suivant une autre caractéristique, lesdits sels phosphoriques ou phosphoreux sont des sels non organiques.

Dans un exemple d'application du procédé suivant l'invention, on a fabriqué 2000 litres de lysat de levures en:
a) chargeant dans un réacteur porté à la température de 72° C, des levures pour un poids équivalent à 1800 kg et du chlorure de sodium pour un poids allant de 46,8 à 180 kg,
b) brassant le mélange obtenu quand il a atteint environ 50° C,
c) arrêtant le brassage et l'apport de chaleur quand le mélange brassé a atteint 72° C,
d) ajoutant, une fois le mélange brassé refroidi, de l'acide phosphorique à raison de 46,8 à 180 kg.

On obtient alors un lysat dont les caractéristiques au litre sont les suivantes:
- taux de protéines (N x 6,25) 12 à 16,5 %
- taux de matières sèches 26 à 32,5 %
- poids spécifique 1,050 à 1,150 kg
   cellules revivifiables au gramme moins de 10 000

On peut transformer ce lysat humide en poudre par séchage en utilisant, par exemple, un séchoir à billes, à turbines ou à buses. Le produit en poudre obtenu a alors les caractéristiques suivantes:
- taux de protéines (N x 6,25) 36 à 48,5 %
- taux de matières sèches 88 à 98 %
- cellules revivifiables au gramme moins de 10 000

Dans l'exemple mentionné ci-dessus, le pourcentage le plus faible, que ce soit de protéines ou de matières sèches, correspond au cas où on utilise de la levure de bière comme produit de départ et le pourcentage le plus élevé au cas où on utilise de la levure de panification de bonne qualité. Selon le mélange utilisé de ces levures ou leur qualité, on obtient un pourcentage intermédiaire.

A noter encore que, dans l'exemple qui vient d'être décrit, on a laissé refroidir le mélange brassé avant d'ajouter l'acide phosphorique, mais cela n'est pas indispensable.

La levure de panification utilisée peut être de la levure dite humide ou de la levure dite sèche. Ce peut être aussi un sous-produit de la fabrication de celles-ci. Le levure de bière est généralement humide.

## Revendications

1. Procédé de préparation de lysat de levures de souche "Saccharomyces cerevisiae", ou de souche "Saccharomyces carlsbergensis" ou d'un mélange de ces deux souches, caractérisé en ce qu'on fait subir aux levures un traitement physico-chimique d'éclatement de la paroi des cellules de levure en chauffant à une température supérieure à 72° C pendant au moins 20 minutes en présence de chlorure de sodium mélangé à la levure à raison de 2,6 à 10 % de chlorure de sodium, puis un second traitement dans lequel le lysat de cellules éclatées et salées de levures est additionné de 2,6 à 10 % d'un sel ou d'un mélange de sels phosphoriques ou phosphoreux, ou d'acide ou d'anhydrique phosphorique ou phosphoreux.

2. Procédé suivant la revendication 1, caractérisé en ce que lesdits sels phosphoriques ou phosphoreux utilisés sont des sels minéraux.

3. Procédé suivant la revendication 1, caractérisé en ce que, pour fabriquer 2000 litres de lysat de levures, on:
a) charge dans un réacteur porté à la température de 72° C, des levures pour un poids équivalent à 1800 kg et du chlorure de sodium pour un poids allant de 46,8 à 180 kg,
b) brasse le mélange obtenu quand il a atteint environ 50° C,
c) arrête le brassage et l'apport de calories quand le mélange brassé a atteint 72° C, et on
d) ajoute, une fois le mélange brassé refroidi, de l'acide phosphorique à raison de 46,8 à 180 kg.

## Claims

1. Method for producing yeast lysates of the "Saccharomyces cerevisiae" or the "Saccharomyces carlsbergensis" family or a mixture of these two families, characterised in that the yeasts are subjected to a physicochemical bursting treatment of the wall of the yeast cells by heating to a temperature higher than 72°C during at least 20 minutes in the presence of sodium chloride mixed with the yeast in a proportion of 2.6 to 10% of sodium chloride, then a second treatment in which 2.6 to 10% of a salt or a mixture of phosphoric or phosphorous, or acid or anhydrous phosphoric or phosphorous, salts is added to the lysate of the burst and salted yeast cells.

2. Method according to claim 1, characterised in that said utilised phosphoric or phosphorous salts are mineral salts.

3. Method according to claim 1, characterised in that, in order to produce 2000 litres of yeast lysates:
a) yeasts for an equivalent weight of 1800 kg and sodium chloride for a weight ranging from 46.8 to 180 kg are filled into a reactor raised to the temperature of 72°C,
b) the obtained mixture is stirred when it reaches approximately 50°C,
c) the stirring and the calorie input is stopped when the stirred mixture has reached 72°C, and
d) the phosphoric acid is added in a proportion of 46.8 to 180 kg once the stirred mixture has cooled.

## Patentansprüche

1. Verfahren zur Herstellung von Hefelysaten des Stammes *"Saccha-* *romyces cerevisiae"* oder des Stammes *"Saccharomyces carlsbergensis*" oder einer Mischung dieser beiden Sorten, dadurch gekennzeichnet, daß man die Hefen einer physiko-chemischen Behandlung zum Bersten der Zellwände der Hefe aussetzt durch Erhitzen innerhalb von 20 Minuten auf eine Höchsttemperatur von 72 °C in Gegenwart von Natriumchlorid, welches mit der Hefe in einer Menge von 2,6 bis 10 % Natriumchlorid gemischt ist, und danach einer zweiten Behandlung, in der dem Lysat der geborstenen und eingesalzenen Hefezellen von 2,6 bis 10 % an einem Salz oder einer Mischung von Salzen der Phosphorsäure oder der phosphorigen Säure oder Phosphorsäure oder phosphorige Säure oder deren Anhydride zugesetzt wird.

2. Verfahren nach Anspruch 1, dadurch gekennzeichnet, daß die besagten benutzten Salze der Phosphorsäure, oder der phosphorigen Säure Mineralsalze sind.

3. Verfahren nach Anspruch 1, dadurch gekennzeichnet, daß man für die Herstellung von 2000 Litern Hefelysat
a) ein Reaktionsgefäß, das auf eine Temperatur von 72 °C gebracht wird, mit Hefe mit einem Äquivalenzgewicht von 1800 kg und Natriumchlorid mit einem passenden Gewicht von 46,8 bis 180 kg beschickt,
b) die Mischung rührt, bis sie ungefähr 50 °C erreicht hat,
c) das Umrühren und die Zufuhr von Wärme beendet, wenn die gerührte Mischung 72 °C erreicht hat, und
d) auf einmal in die gerührte, gekühlte Mischung Phosphorsäure in einer Menge von 46,8 bis 180 kg hineinrührt.
